# EUROPEAN PATENT SPECIFICATION

(11) **EP 4 351 704 B1**
(45) Date of publication and mention of the grant of the patent: **28.05.2025**
(21) Application number: 22732267.4
(22) Date of filing: 10.06.2022
(51) Int. Cl.: A61N 1/05, A61N 1/36, A61N 1/375, A61B 5/263, A61B 5/293

(54) **SYSTEM FOR PERIPHERAL NERVE STIMULATION**
SYSTEM ZUR PERIPHEREN NERVENSTIMULATION
SYSTÈME POUR STIMULATION DES NERFS PÉRIPHÉRIQUES

(30) Priority: 11.06.2021 EP 21382525
(43) Date of publication of application: 17.04.2024
(73) Proprietor: Innervia Bioelectronics SLU, 08028 Barcelona (ES)
(72) Inventor: BAKKER, Jurriaan, 5591 PZ, Heeze (NL); DONEGA, Matteo, 68330 Huningue (FR); RODRIGUEZ, Elisa, 47008 Valladolid (ES)
(74) Representative: DTS Patent- und Rechtsanwälte PartmbB
(86) International application number: PCT/EP2022/065879
(87) International publication number: WO 2022/258824

(56) References cited:
- WO-A1-2020/087123
- US-A1- 2017 007 146

## Description

The present invention relates to a system for peripheral nerve stimulation (PNS), in particular for monitoring of neuronal and/or muscle activity.

In this relation, neuroprosthetic devices are powerful tools to monitor, prevent and treat neural diseases, disorders and conditions by interfacing electrically with the nervous system. They are capable of recording and stimulating electrically neural activity once implanted in the nervous tissue or applied as external devices on the patient's body for interaction with the nervous tissue. Currently, most neuroprosthetic technologies apply electrodes interfacing with neural tissue.

In this context, nerve conduction velocity (NCV) is an important aspect of nerve conduction studies. It describes the speed at which an electrochemical impulse propagates down a neural pathway/structure/fiber. Nerve conduction velocities and how to measure those are, in general, well known in the prior art.

In particular, conduction velocities are affected by a wide range of factors, which include a.o. age, sex and various medical conditions. Studies allow for better diagnoses of various neuropathies, especially demyelinating diseases, as these conditions result in reduced or non-existent conduction velocities.

Conventional NCV measurements use a stimulation pulse on e.g., the forearm, while measuring the response at a certain distance, e.g. at a distance of more than 10 cm.

The velocity is relatively low compared to normal electrical propagation (order of the speed of light), e.g. 0.5 m/s for IV/C sensory fibers and up to 120 m/s for la sensory/alpha motor fibers.

Consequently, measuring the delays between stimulation and responses over a large distance on the skin is very well feasible.

For implanted, miniaturized electrode arrays however, such as nerve cuffsor small skin patches for peripheral nerve stimulation, the distance is much smaller. Hence, the time to detect the delays in the course of the stimulation propagation along the corresponding nerve fibers is much shorter.

This circumstance leads to various issues for NCV measurements with such miniaturized recording arrays/devices, e.g. electrode arrays, which may be summarized as follows:
- High sampling frequency is required.
- Accurate time synchronization between stimulation and recording channels is required.
- Relatively large stimulation intensity could saturate the neighbouring recording electrodes/recording amplifiers/circuitry, causing a "dead-time" that is larger than the latency to be measured.
- The stimulation waveform and its non-infinitely small duration is causing a relatively big error term to the velocity measurement.
- The actual stimulation waveform (and its parameters) used might affect the latency measurement, as the neural activation (onset time, intensity and wave shape) might depend on the applied stimulation waveform.
- It is not the location of the activating/stimulation electrode/device that determines the exact location of activation in the nerve, but a combination of tissue, nerve anatomy and the excitation potential field. Hence, the NCV method is prone to spatial determination errors.
- Minimum three, preferably four (two times differential) wires/recording channels are required per NCV measurement location.

In summary, due to particularly the geometrical restrictions in the case of miniaturized electrode array/devices for specific neurostimulation applications, there are numerous issues which cause the NCV measurement result to be inaccurate and unreliable.

WO2020/087123A1 discloses an implantable stimulator, e.g., an implantable spinal cord stimulator, inter alia adapted for estimating a nerve conduction velocity, said stimulator comprising at least one stimulation electrode and at least one measurement electrode, e.g., a plurality of measurement electrodes, a measurement circuitry for obtaining a neural measurement from the at least one measurement electrode, and a processor.

It is an object of the invention to provide a system for peripheral nerve stimulation which allows for monitoring and/or measurement of neuronal and/or muscle activity, wherein the process and the handling of the measurement as well as of the measurement results is eased and simplified in order to receive more reliable and accurate results, in particular by providing a more secure, stable and reliable type of measurement device along a nerve structure/fiber.

The aforementioned objects are solved by the subject-matters of the independent claim 1. Advantageous configurations of the invention are described in dependent claims.

According to the present invention a system for peripheral nerve stimulation, in particular for detecting and/or monitoring of neuronal and/or muscle activity, comprising at least one neurostimulation device, wherein the neurostimulation device comprises at least a first recording means and a second recording means, wherein the first recording means and the second recording means are configured to be arranged at a recording means distance from one another and respectively configured to detect/measure/determine a stimulus being propagated along a nervous structure, in particular along an efferent nerve and/or an afferent nerve, such that a speed of the stimulus being propagated along the nervous/nerve structure/fiber can be determined.

The present invention is based on the idea to use an (pre-defined) arrangement of recording means, in particular an electrode or coil pattern, with known/defined distances and, optionally, known sensitivity differences, between the electrodes/coils, where those are preferably combined/connected into one common recording channel/connection.

Insofar, the invention can be considered to be basically inspired by the optical Fiber Bragg Grating concerning optical light reflection/transmission/wavelength shifts which have at least some parallels to induced currents in electrodes/coils with respective time-shifts.

Consequently, the propagating electrochemical impulse appears not only once but multiple times on the recording channel timeline/the common recording signal. In this timeline/common recording signal, the relative position between those pulses identifies the exact location of where the pulse appeared on the array/pattern/arrangement of recording means.

Moreover, such (common) recording signals do not only provide the propagation speed, but also e.g. the direction and thus the type of nerve/nervous structure/fiber which propagated the measured stimulus, i.e. efferent or afferent type of structures/sensors/fibers.

According to the present invention the first and second recording means are preferably arranged at a recording means distance.

The recording means distance can be a pre-defined and fixed distance between the first/second recording means.

Alternatively, the distance between the first and second recording means being arranged on the patient's body can be measured and subsequently set, e.g. by corresponding input to a control unit of the system, as the recording means distance.

Moreover, the first/second recording means are configured to determine/detect/measure a stimulus being propagated along a nerve/nervous structure like a nerve fiber. In particular, such stimulus can be based on a natural neural/muscle activity or can be induced by an (external) neural stimulation.

On basis of such recording means distance and the resulting measurement/detection/recording signal of the first and second recording means, a run time of the propagated stimulus along the recording means distance can be determined.

In consequence, the speed of the propagated stimulus can be calculated on basis of the recording means distance and the run time of the propagated stimulus along such recording means distance.

Moreover, such configuration of the system, i.e. of the first/second recording means, also allows to determine further information like the direction of nerve propagation and the kind of nervous structure/fiber propagating the respective stimulus.

Hence, the system according to the present invention provides an easy to use and integral approach on how to proceed NCV measurement by using miniaturized recording/stimulation devices, as applied in PNS.

According to one embodiment of the present invention the first and/or second recording means comprises at least two recording elements, wherein the recording elements of the first recording means are arranged in a first pattern, wherein the recording elements of the second recording means are arranged in a second pattern.

In particular, the first and/or second recording means can comprise two or more recording elements being arranged in a certain pattern. It is also possible that the first recording means comprise more or less recording elements than the second recording means.

In a non-claimed example, the first and second pattern can be identical.

According to the invention, the first and second pattern deviate from each other. In particular, the first/second pattern can deviate in the structural/geometrical embodiment of the respective recording elements, the arrangement of the recording elements of the first/second recording means relative to each other and/or the like.

The first and/or second recordings means can provide a specific signature during recording/detecting/measurement of a stimulus which can allow their identification on the (common) recording signal, as provided by the first/second recording means to e.g. the control unit of the system.

In another preferred embodiment the first pattern comprises a first recording element distance between the recording elements of the first recording element and the second pattern comprises a second recording element distance between the recording elements of the second recording means. In a further preferred embodiment, the first recording element distance and the second recording element distance are identical or deviate from each other.

The first and second pattern of the first/second recording means can preferably deviate from each other by the first/second recording element distance being provided between the at least two recording elements of each of the first/second recording means.

For example, the recording element distance between recording elements of the second recording means can be larger than in case of the first recording means.

Such different recording elements distances can allow for a specific signature of the first and second recording means. Thus, on basis of the (common) recording signal of the determined nerve stimulus the first and second recording means, comprising individual patterns of recording elements, can be identified respectively, namely due to their unique, specific signature.

Such signature, e.g. by the first/second patterns of the first/second recording means, can, for example, provide an easier and beneficial signal processing.

Preferably, the first pattern and second pattern preferably deviate from each other only with respect to the first and second recording element distance.

Accordingly, on basis of the resulting (common) recording signal, as forwarded by the first/second recording means, in particular forwarded by the recording elements of the first/second recording means, the first/second recording means can be easily identified.

In this regard, the first recording means and the second recording means provide a unique signature on the (common) recording signal, which reflects the propagation of the detected nerve stimulus, due to the different first/second recording elements distances being provided between their respective recording elements.

Moreover, only one type of recordings elements has to be manufactured and provided for the system, whereby only the respective recording element distances are changed within the first/second recording means of the system.

Hence, an easy and cost efficient manufacturing of the system according to the present invention can be provided.

According to one preferred embodiment each recording element is provided in form of at least one electrode or coil.

For example, the first/second recording means, i.e. the respective recording elements, can be implemented as recording electrodes in a neurostimulation device for PNS.

Alternatively, the first/second recording means, i.e. the respective recording elements, can be provided in form of recording coils, e.g. in a corresponding nerve cuff for PNS.

In this context, the neurostimulation device can be provided as an electrode device or as a coil device.

The system, in particular the corresponding recording means, can be implemented in various different ways as well as in an efficient and appropriate manner for the respective field of application.

In one embodiment of the present invention the neurostimulation device comprises at least one stimulation means for transmitting stimulation signals to a patient's tissue in order to initiate neuronal and/or muscle stimulation, preferably being configured in form of at least one electrode or coil.

The neurostimulation device of the system according to the present invention can be provided as an electrode device comprising at least one stimulation electrode and at least two recording means, preferably each comprising at least two electrodes as recording elements being arranged in a first/second pattern.

Alternatively, the stimulations means can be implemented as a stimulation coil, preferably in combination with recording means, i.e. recording elements, being implemented in form of coils as well.

As a further alternative, the stimulation means can be provided in form of at least one electrode whereby the recoding elements are provided in form of at least one coil respectively, or vice versa.

The system, comprising the neurostimulation device having at least one stimulation means, can be used in applications like deep brain stimulation or peripheral nerve stimulation. Due to the specific implementation of the recording means, the system can be further used, alternatively or in parallel to the stimulation application itself, for NCV measurements.

Moreover, the system can be used to detect/measure/determine a natural neural activity as well as a (neuro-)stimulation induced activity can be provided via the stimulation means for execution of an NCV measurement.

According to another embodiment the first recording means and the second recording means, in particular recording elements of the first recording means and recording elements of the second recording means, comprise different recording sensitivities.

The recording elements of one of the recording means can comprise same or different recording sensitivities. Alternatively, the recording sensitivities of the recording elements of one recording means can be equal, while recording sensitivities of the first and second recording means differ. Moreover, it is also possible that each recording element of the system comprises a different/unique recording sensitivity.

In particular, the recording/measurement/detection signal as forwarded by the recording means, i.e. the respective recording elements, can differ in case of one stimulus being detected/determined due to the different recording sensitivities.

In this context, the first/second recording means can provide different signatures, not only with respect to time shifts within the (common) recording signal, but also with respect to the intensity of the recording signal being provided by the recording means due to their recording sensitivity.

By such different recording sensitivities a further signature of the respective recording means within the recording signals, being forwarded by the recording means, is provided.

In another embodiment the system further comprises a control unit being configured to operate, in particular to control and/or regulate the operation of, the neurostimulation device and/or to provide signal processing of the signals as received from the first and second recording means.

The control unit can receive the (common) recording signal(s) from the first/second recording means for subsequent signal processing. Preferably, the control unit can be configured to analyze the recording signal in order to determine measured/detected stimulus along the recording signal and/or to determine a time between the detection of the stimulus by the first and second recording means.

In particular, the control unit can calculate the nerve conducting velocity based on the (common) recording signal(s) received.

Moreover, the control unit can be provided in combination with input means and output means of the system, e.g. to allow a user to provide input concerning the recording means distance and/or the first/second recording element distance. Via the output means, the control unit can provide the results of the NCV measurement to the user, namely the speed of the propagated stimulus as measured/detected by the recording means.

In one further preferred embodiment of the invention a recording signal from the first and/or second recording means, in particular of the recording elements of the first and/or second recording means, is forwarded via a common signal connection, preferably to a control unit of the system.

**In** particular, the recording signals of the single recording elements can be combined to one single, common signal connection. **In** this context, the recording signals can be considered to be combined to a common recording signal.

Preferably the common signal connection can be provided as a (data) communicative and/or electrical connection between the recording means, i.e. the recording elements, to the control unit of the system, a data storage, a communication device for further forwarding of the recording signal, to an external server or the like.

Moreover, means for combining the recording signals of the single recording elements of the first/second recording means to a combined recording signal can be provided in order to forward a single combined recording signal via the common signal connection.

By the common signal connection the infrastructure of the system, in particular in the vicinity of the patient and with regard to the arrangement of the recording means, can be simplified. Furthermore, the size of the overall system, in particular of the neurostimulation device can be reduced.

Moreover, by forwarding a common recording signal via one common signal connection the subsequent signal processing can be focused on the single signal, whereby the unique configuration of the at least two recording means ensure the appropriate identification of the respectively recorded stimulus.

In particular, the need and adjustment of (neurostimulation) therapy, either by control or regulation of the system, e.g. in terms of a closed loop system, can be assessed by utilization of the present invention, for example in context of (but not limited to) these applications.

In summary, the system according to the present invention particularly allows in a beneficial manner and in the specific context of miniaturized neurostimulation devices/arrays
- to detect nerve activity;
- to measure its propagation direction;
- to measure its propagation speed and hence nerve conduction velocity;
- to measure nerve conduction based on natural neural activity and/or (electrical, optical, ultrasound, and/or other types of) stimulation induced activity; and/or
- to reduce the size/footprint of the device by reducing the amount of conducting wires/recording channels per NCV measurement.

In summary, the system according to the present invention can be specifically configured to be used for PNS applications, in particular for invasive (e.g. in form of microneedle devices) or transcutaneous (e.g. in form of skin patches) sensors for nerve conduction velocity (NCV) measurement in the context of PNS applications.

Further details and advantages of the present invention shall now be disclosed in the connection with the enclosed drawings.

It is shown in:
- **Fig. 1**: a schematic illustration of alternative embodiments of a system comprising a neurostimulation device in form of a nerve cuff;
- **Fig. 2**: a schematic illustration of alternative embodiments of a system comprising a neurostimulation device in form of a skin patch;
- **Fig. 3**: a schematic illustration of alternative embodiments of a system comprising a neurostimulation device in form of a percutaneous device and a paddle device;
- **Fig. 4**: a schematic illustration of a first embodiment concerning the measurement principle as applicable by embodiments according to Fig. 1 and/or Fig. 2; and
- **Fig. 5**: a schematic illustration of a second embodiment concerning the measurement principle as applicable by embodiments according to Fig. 1 and/or Fig. 2.

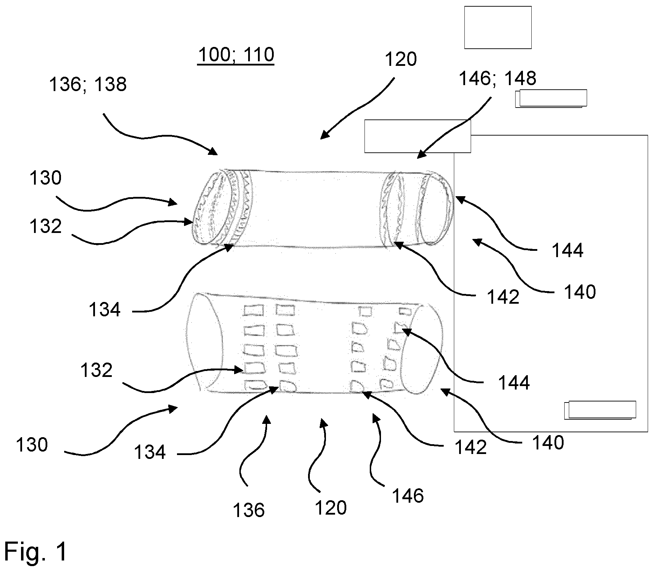

In **Fig. 1** a schematic illustration of alternative embodiments of a system 100 comprising a neurostimulation device 110 in form of a nerve cuff is shown. In particular, two alternatives of a neurostimulation device 110 in form of a nerve cuff are illustrated.

According to Fig. 1 the neurostimulation device 110 comprises a first recording means 130 and a second recording means 140.

The first and second recording means 130; 140 are arranged in a recording means distance 120 from each other.

The first recording means 130 is provided with two recording elements 132; 134 being arranged in a first pattern 136.

The recording elements 132; 134 of the first recording means 130 are arranged in a first recording element distance 138 from each other.

The second recording means 140 is provided with two recording elements 142; 144 being arranged in a second pattern 146.

The recording elements 142; 144 of the second recording means 140 are arranged in a second recording element distance 148 from each other.

According to Fig. 1 the first recording element distance 138 differs from the second recording element distance 148. In particular, the second recording element distance 148 is larger than the first recording element distance 138.

According to the two alternatives shown in Fig. 1, the recording elements 132; 134; 142; 144 can be provided in form of at least one coil (upper illustration of Fig. 1) or in form of at least one electrode (lower illustration of Fig. 1) respectively.

Preferably, the recording elements 132; 134; 142; 144 can each be formed as one coil.

Alternatively, each recording element 132; 134; 142; 144 can be provided in form of multiple electrodes, as shown in Fig. 1.

For example, the multiple electrodes of each recording element 132; 134; 142; 144 can be provided and distributed along the circumference of the neurostimulation device 110 in form of a nerve cuff, according to Fig. 1.

The material for the electrodes can be at least partially graphene, especially reduced graphene oxide (RGO).

In one embodiment, but also in all embodiments disclosed in this disclosure, the electrode device has at least one electrode comprising graphene, in particular being made of graphene or a graphene-based material or comprising a graphene (based) coating.

Preferably, different forms of graphene (based) materials may be used in the context of the present invention, like e.g. reduced graphene oxide (rGO), graphene oxide, chemical vapour deposited graphene (CVD Graphene) or any other potential form of graphene.

In particular, such graphene based materials can provide improved electrical and mechanical properties, e.g. a beneficial flexibility of the resulting electrode.

Such graphene electrodes particularly provide higher safe charge injection capacity as well as the signal-to-noise ratio/performance can be improved. Thereby, the electrode size can be reduced, even if the same amount of electrodes is maintained.

Hence, along a cross-section of the electrode device the cross-sectional area of the at least one electrode can be reduced.

Moreover, such graphene-based electrodes can provide for a safe electrical interface in aqueous environments, like in the context of neuromodulation of nervous tissue.

By the recording elements 132; 134; 142; 144 of the first and second recording means 130; 140 the first pattern 136 and the second pattern 146 is formed.

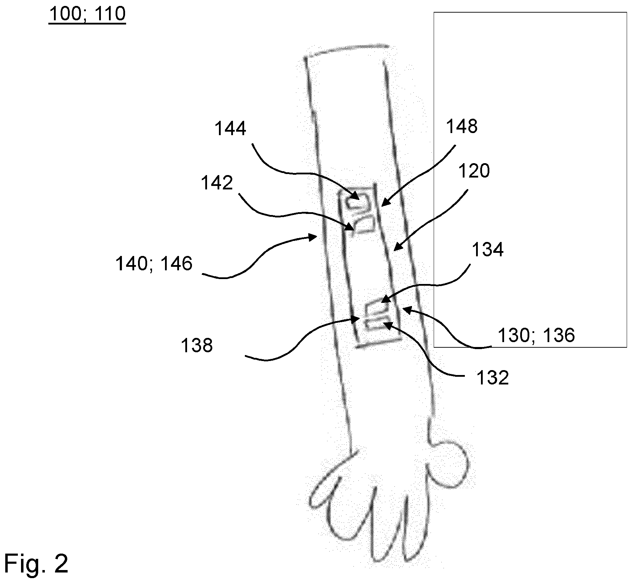

In **Fig. 2** a schematic illustration of alternative embodiments of a system 100 comprising a neurostimulation device 110 in form of a skin patch, in particular for PNS applications, is shown.

The neurostimulation device 110 is provided with a first recording means 130 and a second recording means 140 being separated from each other by a recording means distance 120 along the extension of the neurostimulation device 110.

The recording elements 132; 134 of the first recording means 130 are arranged in a first pattern 136, wherein the recording elements 142; 144 of the second recording means 140 are arranged in a second pattern 146.

In particular, the neurostimulation device 110 in form of a skin patch may be provided with a rectangular shape, as shown in Fig. 2, in order to allow for an increased recording means distance 120 between the first and second recordings means 130; 140.

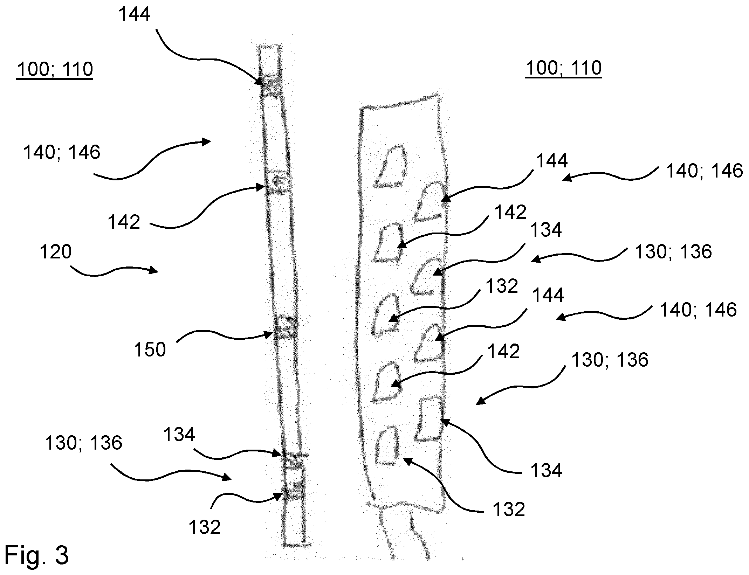

In **Fig. 3** a schematic illustration of alternative embodiments of a system comprising a neurostimulation device 110 in form of a percutaneous device (left illustration of Fig. 3) and in form of a paddle device (right illustration of Fig. 3) are shown.

In particular, the percutaneous neurostimulation device 110 may be formed in a strip-like manner (left illustration of Fig. 3).

Alternatively, the neurostimulation devices 110, like the percutaneous device 110 shown in Fig. 3, can be provided in any geometry and form being applicable and suitable for PNS.

According to Fig. 3 the neurostimulation device 110 comprises the first recording means 130 and the second recording means 140.

The first and second recording means 130; 140 are arranged in the recording means distance 120 from each other.

The first recording means 130 is provided with two recording elements 132; 134 being arranged in the first pattern 136.

The recording elements 132; 134 of the first recording means 130 are arranged in the first recording element distance 138 from each other.

The second recording means 140 is provided with two recording elements 142; 144 being arranged in the second pattern 146.

The recording elements 142; 144 of the second recording means 140 are arranged in the second recording element distance 148 from each other.

According to the alternatives as shown in Fig. 3, the recording elements 132; 134; 142; 144 can be provided in form of at least one electrode respectively. Alternatively, the recording elements 132; 134; 142; 144 may be provided in form of coils.

By the recording elements 132; 134; 142; 144 of the first and second recording means 130; 140 the first pattern 136 and the second pattern 146 are provided/formed.

As a further alternative in the context of the present invention, electrodes/coils can be distributed along the circumference/extension of a neurostimulation device 110 in order to form the recording elements 132; 134; 142; 144 respectively.

Moreover, Fig. 3 shows the embodiment of a percutaneous device 110 with an additional stimulation means 150 (left illustration of Fig. 2) as well as an embodiment (right illustration of Fig. 2) which is particularly formed for the purpose of NCV measurement, thus without an additional stimulation means 150.

In particular, the stimulation means 150 can be provided inbetween the recoding means distance 120 of the neurostimulation device 110, thus between the first and second recording means 130; 140.

In the context of Fig. 3, the stimulation means 150 is provided in the same form as the recording elements 132; 134; 142; 144, thus in form of an electrode.

Alternatively, the stimulation means 150 can be provided in form of at least one coil.

Moreover, it is also possible that the recording elements 132; 134; 142; 144 are provided e.g. as coils, while the stimulation means 150 is provided in form of an electrode.

Furthermore, the at least one stimulation means 150 can be arranged at another location of the stimulation device 110 as well, e.g. at a top or bottom of the neurostimulation device 110 .

Moreover, the further embodiment according to Fig. 3 in form of a paddle device 110 (right illustration of Fig. 3) particularly shows an implementation with multiple first and second recording means 130; 140.

In particular, first and second recording means 130; 140 may be arranged multiple times and in an alternating manner.

By providing the first recording means 130 and the second recording means 140 with a specific signature respectively, like different first/second recording element distances 138; 148 or different sensitivity properties, the determination of a propagation of a nerve stimulus along the extension of the neurostimulation device 110 of the system 100 can be further enhanced.

In this context the first pattern 138 of the first recording means 130 and the second pattern 148 of the second recording means 140 may provide a common and/or homogenous pattern of recording elements 132; 134; 142; 144, in particular of repetitively arranged recording elements 132; 134; 142; 144.

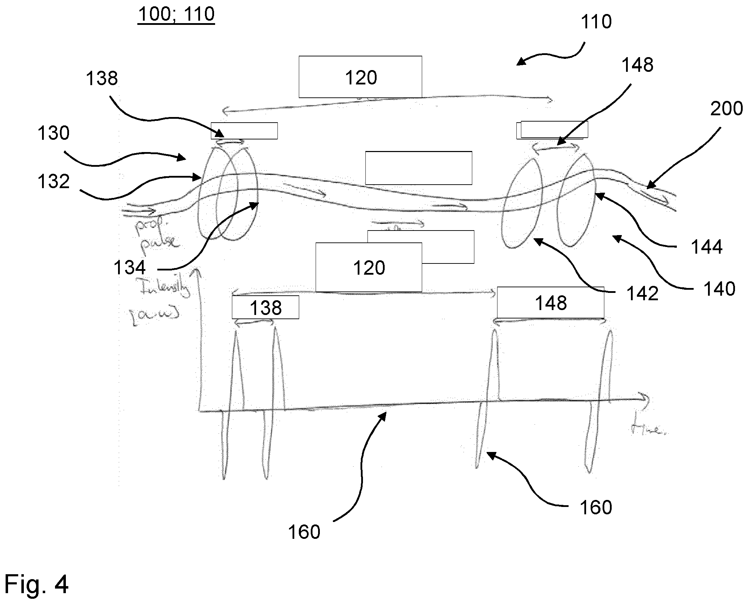

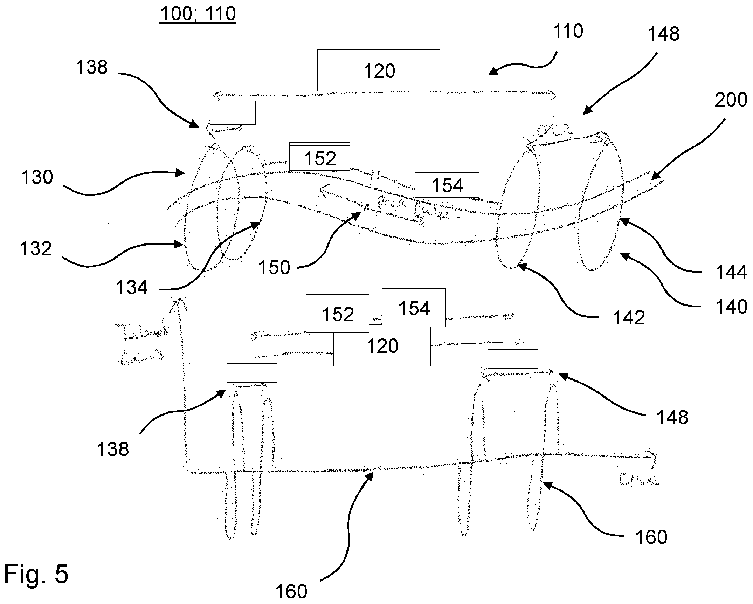

In **Fig. 4** **and** **Fig. 5** a schematic illustration of alternative embodiments concerning the measurement principle as applicable by embodiments according to Fig. 1 and/or Fig. 2 and/or Fig. 3 are shown. In particular, the function of the system 100 having a neurostimulation device 110, as exemplarily shown in Fig. 1 to Fig. 3, can be described as follows.

In Fig. 4 a stimulus/nerve pulse is propagated along a nerve structure/fiber 200.

Along the nerve 200 the first and second recording means 130; 140 are arranged, e.g. in form of coils being provided on a nerve cuff-like neurostimulation device 110 of the system 100.

The first and second recording means 130; 140 are separated from each other by the recording means distance 120.

Moreover, the first recording means 130 forms a first pattern 136 with two recording elements 132; 134 being separated by the first recording element distance 138.

The second recording means 140 form the second pattern 146 with two recording elements 142; 144 being separated by the second recording element distance 148.

When the propagating stimulus is detected by one of the recording elements 132; 134; 142; 144 a recording signal is provided, indicating an intensity peak over the time(-line), as illustrated at the lower diagram of Fig. 4.

In particular, all recording elements 132; 134; 142; 144 can be connected to e.g. a control unit of the system 100 via a common signal line.

Thus, the recording signals of the several recording elements 132; 134; 142; 144 can preferably be combined to a common recording signal 160, as shown in Fig. 4.

As the natural nerve stimulus or an induced nerve stimulus, e.g. induced by the stimulations means 150, propagates further along the nerve fiber 200, it is detected/determined/measured by each of the recording elements 132; 134; 142; 144 of the first and second recording means 130; 140.

According to the embodiment of Fig. 4, the resulting common recording signal 160 indicates four intensity peaks over the time(line), each being provided/determined by one of the corresponding recording elements 132; 134; 142; 144.

The height of the intensity peak in the corresponding diagram of the common recording signal depends on the (recording) sensitivity of the respective recording elements 132; 134; 142; 144.

As can be seen from Fig. 4, the configuration of the neurostimulation device 110, e.g. with respect to the recording means distance 120 as well as the first/second recording elements distances 138; 148, is reflected and can be gathered from the diagram visualizing the common recording signal 160.

According to Fig. 4, the recording means distance 120 can describe the distance between the beginning of the first recording means 130 to the beginning of the second recording means 140.

Moreover, according to Fig. 4, the recording elements 132; 134; 142; 144 are identically configured, in particular comprise the same sensitivity properties. This can particularly be gathered form the equal height of the peaks of the common recording signal 160.

Alternatively, each of the recording elements 132; 134; 142; 144 can have a different sensitivity which can cause different peak heights within the common recording signal 160.

In particular, the recording elements of a recording means 130; 140 can have same or different sensitivities, providing a sensitivity profile, i.e. a signature, for each recording means 130; 140.

The difference between the first recording means 130 and the second recording means 140 according to Fig. 4 is the distance between the respective recording elements 132; 134; 142; 144, hence a deviation between the first recording element distance 138 and the second recording element distance 148.

The first pattern 136 and the second pattern 146 differ from each other with regard to the first/second recording element distance 138; 148.

In Fig. 5 an embodiment according to Fig. 1 to Fig. 2 is shown, in particular with a stimulation means 150 as shown in Fig. 3.

According to Fig. 5 the stimulation means 150 is situated between the first and second recording means 130; 140.

The stimulation means 150 can induce a nerve stimulation in order to initiate a propagating stimulus along the nerve 200.

The arrangement of the stimulation means 150 in Fig. 5 provides a first stimulation means distance 152 and a second stimulation means distance 154.

In consideration of the first and second stimulation means distances 152; 154 the common recording signal 160 can indicate the propagation of the stimulus along the nerve 200 over the time, in particular subsequent to a signal processing by the control unit.

In summary, the system according to the present invention can provide a NCV measurement for miniaturized neurostimulation devices 110, like neurostimulation devices 110 for PNS.

In particular, a natural nerve stimulus or an induced nerve stimulus, i.e. provided via the stimulation means 150, propagating along a nerve 200 can be determined via the neurostimulation device 110.

Thereby, the NCV measurement and signal (post-)processing, particularly in the context of such miniaturized systems 100, can be simplified by utilizing first and second recording means 130; 140 being situated/arranged within a (pre-defined) recording means distance 120.

Moreover, by combining the recording signals of the recording means 130; 140, in particular of the associated recording elements 132; 134; 142; 144, to a common signal connection, the wiring of the neurostimulation device 110 can be simplified and the size of the system 100, in particular of the neurostimulation device 110, can be reduced, as well as a single common recording signal 160 can be provided for further processing.

### Reference numerals

- 100: System
- 110: Neurostimulation device
- 120: Recording means distance
- 130: First recording means
- 132: Recording element
- 134: Recording element
- 136: First pattern
- 138: First recording element distance
- 140: Second recording means
- 142: Recording element
- 144: Recording element
- 146: Second pattern
- 148: Second recording element distance
- 150: Stimulation means
- 152: First stimulation means distance
- 154: Second stimulation means distance
- 160: Common recording signal
- 200: Efferent/afferent nerve

## Claims

1. System (100) for peripheral nerve stimulation, in particular for detecting and/or monitoring of neuronal and/or muscle activity, comprising at least one neurostimulation device (110),
wherein the neurostimulation device (110) comprises at least a first recording means (130) and a second recording means (140),
wherein the first recording means (130) and the second recording means (140) are configured to be arranged at a recording means distance (120) from one another and
respectively configured to detect a stimulus being propagated along a nervous structure, in particular along an efferent nerve and/or an afferent nerve, such that a speed of the stimulus being propagated along the nervous structure can be determined, wherein
each of the first and second recording means (130; 140) comprise at least two recording elements (132; 134; 142; 144),
wherein the recording elements (132; 134) of the first recording means (130) are arranged in a first pattern (136),
wherein the recording elements (142; 144) of the second recording means (140) are arranged in a second pattern (146) different from the first pattern (136).

2. System (100) according to claim 1,
**characterized in that**
the first pattern (136) comprises a first recording element distance (138) between the recording elements (132; 134) of the first recording element (130) and the second pattern (146) comprises a second recording element distance (148) between the recording elements (142; 144) of the second recording means (140).

3. System (100) according to claim 2,
**characterized in that**
the first recording element distance (138) and the second recording element distance (148) are identical or deviate from each other.

4. System (100) according to one of claims 1 to 3,
**characterized in that**
each recording element (132; 134; 142; 144) is provided in form of at least one electrode or coil.

5. System (100) according to one of the preceding claims,
**characterized in that**
the neurostimulation device (110) comprises at least one stimulation means (150) for transmitting stimulation signals to a patient's tissue in order to initiate neuronal and/or muscle stimulation, preferably being configured in form of at least one electrode or coil.

6. System (100) according to one of the preceding claims,
**characterized in that**
the first recording means (130) and the second recording means (140), in particular recording elements (132; 134) of the first recording means (130) and recording elements (142; 144) of the second recording means (140), comprise different recording sensitivities.

7. System (100) according to one of the preceding claims,
**characterized in that**
the system further comprises a control unit being configured to operate, in particular to control and/or regulate the operation of, the neurostimulation device (110) and/or to provide signal processing of the signals as received from the first and second recording means (130; 140).

8. System (100) according to one of the preceding claims,
**characterized in that**
a recording signal from the first and/or second recording means (130; 140), in particular of the recording elements (132; 134; 142; 144) of the first and/or second recording means (130; 140), is forwarded via a common signal connection, preferably to a control unit of the system (100).

## Patentansprüche

1. System (100) zur peripheren Nervenstimulation, insbesondere zum Erfassen und/oder zum Überwachen einer neuronalen Aktivität und/oder Muskelaktivität, das mindestens eine Neurostimulationsvorrichtung (110) aufweist,
wobei die Neurostimulationsvorrichtung (110) mindestens eine erste Aufzeichnungseinrichtung (130) und eine zweite Aufzeichnungseinrichtung (140) aufweist,
wobei die erste Aufzeichnungseinrichtung (130) und die zweite Aufzeichnungseinrichtung (140) dafür ausgelegt sind, unter einer Aufzeichnungseinrichtungsdistanz (120) zueinander angeordnet zu sein, und jeweils dafür ausgelegt sind, einen Stimulus zu erfassen, der sich entlang einer Nervenstruktur, insbesondere entlang eines efferenten Nervs und/oder eines afferenten Nervs, ausbreitet, so dass eine Geschwindigkeit des sich entlang der Nervenstruktur ausbreitenden Stimulus bestimmt werden kann,
wobei die erste und die zweite Aufzeichnungseinrichtung (130; 140) jeweils mindestens zwei Aufzeichnungselemente (132; 134; 142; 144) aufweisen,
wobei die Aufzeichnungselemente (132; 134) der ersten Aufzeichnungseinrichtung (130) in einem ersten Muster (136) angeordnet sind,
wobei die Aufzeichnungselemente (142; 144) der zweiten Aufzeichnungseinrichtung (140) in einem zweiten Muster (146) angeordnet sind, das sich vom ersten Muster (136) unterscheidet.

2. System (100) nach Anspruch 1,
**dadurch gekennzeichnet, dass**
das erste Muster (136) eine erste Aufzeichnungselementdistanz (138) zwischen den Aufzeichnungselementen (132; 134) des ersten Aufzeichnungselements (130) und das zweite Muster (146) eine zweite Aufzeichnungselementdistanz (148) zwischen den Aufzeichnungselementen (142; 144) der zweiten Aufzeichnungseinrichtung (140) aufweist.

3. System (100) nach Anspruch 2,
**dadurch gekennzeichnet, dass**
die erste Aufzeichnungselementdistanz (138) und die zweite Aufzeichnungselementdistanz (148) identisch sind oder voneinander abweichen.

4. System (100) nach einem der Ansprüche 1 bis 3,
**dadurch gekennzeichnet, dass**
jedes Aufzeichnungselement (132; 134; 142; 144) in Form von mindestens einer Elektrode oder Spule vorgesehen ist.

5. System (100) nach einem der vorhergehenden Ansprüche,
**dadurch gekennzeichnet, dass**
die Neurostimulationsvorrichtung (110) mindestens eine Stimulationseinrichtung (150) zum Übertragen von Stimulationssignalen auf ein Gewebe eines Patienten aufweist, um eine neuronale Stimulation und/oder Muskelstimulation zu initiieren, die vorzugsweise in Form von mindestens einer Elektrode oder Spule ausgelegt ist.

6. System (100) nach einem der vorhergehenden Ansprüche,
**dadurch gekennzeichnet, dass**
die erste Aufzeichnungseinrichtung (130) und die zweite Aufzeichnungseinrichtung (140), insbesondere die Aufzeichnungselemente (132; 134) der ersten Aufzeichnungseinrichtung (130) und die Aufzeichnungselemente (142; 144) der zweiten Aufzeichnungseinrichtung (140), unterschiedliche Aufzeichnungsempfindlichkeiten aufweisen.

7. System (100) nach einem der vorhergehenden Ansprüche,
**dadurch gekennzeichnet, dass**
das System darüber hinaus eine Steuereinheit aufweist, die dafür ausgelegt ist, die Neurostimulationsvorrichtung (110) zu betreiben, insbesondere deren Betrieb zu steuern und/oder zu regeln, und/oder eine Signalverarbeitung der Signale, wie sie von der ersten und zweiten Aufzeichnungseinrichtung (130; 140) empfangen werden, bereitzustellen.

8. System (100) nach einem der vorhergehenden Ansprüche,
**dadurch gekennzeichnet, dass**
ein Aufzeichnungssignal von der ersten und/oder zweiten Aufzeichnungseinrichtung (130; 140), insbesondere der Aufzeichnungselemente (132; 134; 142; 144) der ersten und/oder zweiten Aufzeichnungseinrichtung (130; 140), über eine gemeinsame Signalverbindung vorzugsweise an eine Steuereinheit des Systems (100) weitergeleitet wird.

## Revendications

1. Système (100) de stimulation des nerfs périphériques, notamment de détection et/ou de surveillance de l'activité neuronale et/ou musculaire, comprenant au moins un dispositif de neurostimulation (110) ;
dans lequel le dispositif de neurostimulation (110) comprend au moins un premier moyen d'enregistrement (130) et un second moyen d'enregistrement (140) ;
dans lequel le premier moyen d'enregistrement (130) et le second moyen d'enregistrement (140) sont configurés pour être agencés à une certaine distance de moyen d'enregistrement (120) l'un de l'autre et sont respectivement configurés pour détecter un stimulus propagé le long d'une structure nerveuse, notamment le long d'un nerf efférent et/ou d'un nerf afférant, de façon à pouvoir déterminer une vitesse de propagation du stimulus le long de la structure nerveuse, dans lequel :
chacun des premier et second moyens d'enregistrement (130 ; 140) comprend au moins deux éléments d'enregistrement (132 ; 134 ; 142 ; 144),
dans lequel les éléments d'enregistrement (132 ; 134) du premier moyen d'enregistrement (130) sont agencés dans un premier motif (136),
dans lequel les éléments d'enregistrement (142 ; 144) du second moyen d'enregistrement (140) sont agencés dans un second motif (146) différent du premier motif (136).

2. Système (100) selon la revendication 1,
**caractérisé en ce que :**
le premier motif (136) comprend une première distance d'élément d'enregistrement (138) entre les éléments d'enregistrement (132 ; 134) du premier élément d'enregistrement (130) et le second motif (146) comprend une seconde distance d'élément d'enregistrement (148) entre les éléments d'enregistrement (142 ; 144) du second moyen d'enregistrement (140).

3. Système (100) selon la revendication 2,
**caractérisé en ce que :**
la première distance d'élément d'enregistrement (138) et la seconde distance d'élément d'enregistrement (148) sont identiques ou varient l'une par rapport à l'autre.

4. Système (100) selon l'une quelconque des revendications 1 à 3,
**caractérisé en ce que :**
chaque élément d'enregistrement (132 ; 134 ; 142 ; 144) est prévu sous la forme d'au moins une électrode ou d'une bobine.

5. Système (100) selon l'une quelconque des revendications précédentes,
**caractérisé en ce que :**
le dispositif de neurostimulation (110) comprend au moins un moyen de stimulation (150) pour transmettre des signaux de stimulation au tissu d'un patient afin de réaliser une stimulation neuronale et/ou musculaire, configurée de préférence sous la forme d'au moins une électrode ou bobine.

6. Système (100) selon l'une quelconque des revendications précédentes,
**caractérisé en ce que :**
le premier moyen d'enregistrement (130) et le second moyen d'enregistrement (140), notamment des éléments d'enregistrement (132 ; 134) du premier moyen d'enregistrement (130) et des éléments d'enregistrement (142 ; 144) du second moyen d'enregistrement (140), comprennent différentes sensibilités d'enregistrement.

7. Système (100) selon l'une quelconque des revendications précédentes,
**caractérisé en ce que :**
le système comprend en outre une unité de commande configurée pour actionner, notamment pour commander et/ou réguler le fonctionnement du dispositif de neurostimulation (110) et/ou pour fournir un traitement de signal des signaux tels que reçus des premier et second moyens d'enregistrement (130 ; 140).

8. Système (100) selon l'une quelconque des revendications précédentes,
**caractérisé en ce que :**
un signal d'enregistrement provenant des premier et/ou second moyens d'enregistrement (130 ; 140), notamment des éléments d'enregistrement (132 ; 134 ; 142 ; 144) des premier et/ou second moyens d'enregistrement (130 ; 140), est transféré via une connexion de signal commune, de préférence à une unité de commande du système (100).
